(19) Europäisches Patentamt
European Patent Office
Office européen des brevets

(11) Publication number: **0 021 836**
**B1**

# (12) EUROPEAN PATENT SPECIFICATION

(45) Date of publication of patent specification: **11.05.83**

(21) Application number: **80302153.4**

(22) Date of filing: **26.06.80**

(51) Int. Cl.³: **C 07 D 498/04,**
A 61 K 31/42,
A 61 K 31/43,
A 61 K 31/545
//(C07D498/04, 263/00,
205/00), (A61K31/43, 31/42),
(A61K31/545, 31/42)

## (54) Clavulanic acid derivatives, process for their preparation and pharmaceutical compositions containing them.

(30) Priority: **02.07.79 GB 7922886**

(43) Date of publication of application:
**07.01.81 Bulletin 81/1**

(45) Publication of the grant of the patent:
**11.05.83 Bulletin 83/19**

(84) Designated Contracting States:
**AT BE CH DE FR GB IT LI LU NL SE**

(56) References cited:
**EP - A - 0 007 717**
**DE - A - 2 646 003**

(73) Proprietor: **BEECHAM GROUP PLC**
**Beecham House Great West Road**
**Brentford Middlesex (GB)**

(72) Inventor: **Stirling, Irene**
**38 Harrison Close**
**Reigate Surrey (GB)**
Inventor: **Clarke, Brian Peter**
**'Coneybury' Drive Spur Forest Drive**
**Kingswood Surrey (GB)**

(74) Representative: **Hesketh, Alan, Dr. et al,**
**European Patent Attorney Beecham**
**Pharmaceuticals Great Burgh Yew Tree Bottom Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Clavulanic acid derivatives, process for their preparation and pharmaceutical compositions containing them

The present invention relates to β-lactam antibacterial agents, to the process for their preparation and to compositions containing them.

British Patent Application No. 16764/77 (corresponding to U.S. Serial No. 896441, and Belgian Patent No. 187034), discloses *inter alia* the compounds of the formula (I):

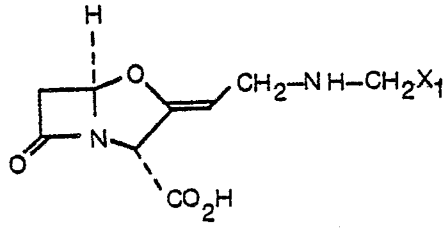

(I)

and esters thereof wherein $X_1$ is a hydrogen atom, or an alkyl group of up to 5 carbon atoms, an alkenyl group of up to 5 carbon atoms, a hydroxy alkyl group of up to 5 carbon atoms or an optionally substituted phenyl group. Such compounds were described as antibacterial agents and β-lactamase inhibitors.

It has now been discovered that stable, crystalline secondary amines can be prepared that are β-lactamase inhibitors that enhance the effectiveness of penicillins or cephalosporins and which also have antibacterial properties in their own right and which are advantageously water soluble.

The present invention provides a compound of the formula (II), or an ester or pharmaceutically acceptable acid addition salt of such an ester;

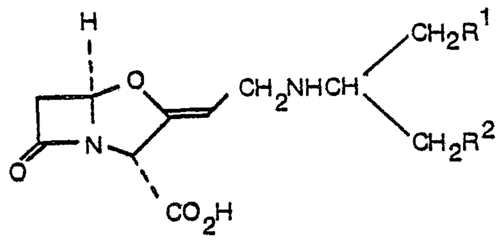

(II)

wherein $R^1$ is a hydrogen atom or a lower alkyl group and $R^2$ is a hydrogen atom or a lower alkyl group; or $R^1$ and $R^2$ together may represent a bond or a group $-(CH_2)_n-$, where n is an integer from 1 to 6.

When used herein the term "lower alkyl" means an alkyl group of 1 to 4 carbon atoms. Thus it will be apparent that suitable values of $R^1$ and $R^2$, which may be the same or different include hydrogen, methyl, ethyl, isopropyl, n-propyl and n-butyl.

Certain favoured moieties $R^1$ are hydrogen, methyl, ethyl, n-propyl and isopropyl.

Certain favoured moieties $R^2$ are hydrogen, methyl, ethyl, n-propyl and isopropyl.

Favourably $R^1$ and $R^2$ together may represent a bond or a group $-(CH_2)_n-$ where n is an integer from 1 to 3.

A particularly preferred compound is 9-N-isopropylaminodeoxyclavulanic acid.

Further preferred compounds are 9-N-cyclopentylaminodeoxyclavulanic acid, 9-N-cyclopropylaminodeoxyclavulanic acid and 9-N-cyclohexylaminodeoxyclavulanic acid.

The compounds of this invention are preferably in the form of the free acid of formula (II) and as such normally exist in the form of a zwitterion, that is they may be represented as shown in formula (IIa):

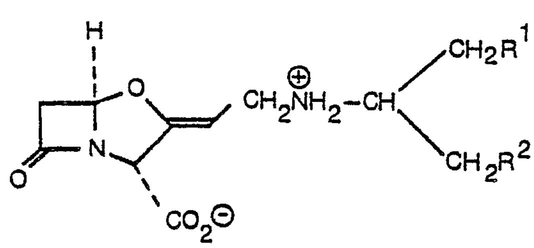

(IIa)

These zwitterionic forms of the compounds are favoured in view of their crystalline form, stability and good solubility.

Esters of the compounds of the formula (II) also form part of this invention, for example as the free base or as the acid addition salt since such compounds can also be used to enhance the effectiveness of penicillins or cephalosporins.

Suitable esters of the compounds of the formula (II) include those of the formulae (III) and (IV):

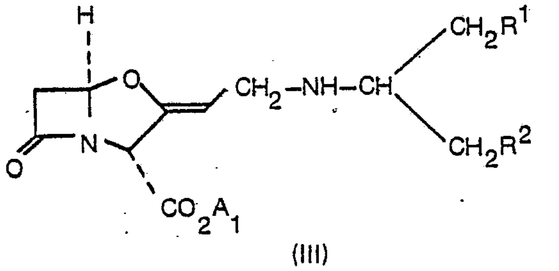

(III)

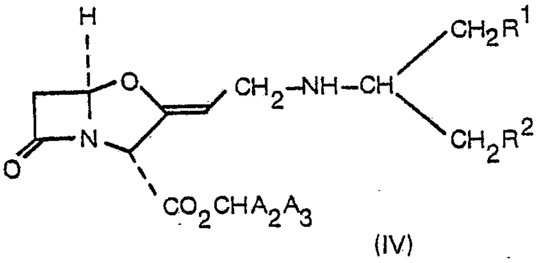

(IV)

wherein $A_1$ is $C_{1-6}$ alkyl optionally substituted by alkoxy, aryloxy, alkoxycarbonyloxy, acyloxy, aralkanoyl, alkanoyl or benzoyl of up to 8 carbon atoms, any phenyl groups in such substituents being themselves optionally substituted by a fluorine, bromine, chlorine, nitro, $C_{1-4}$ alkyl or $C_{1-4}$ alkoxy; or is a phthalidyl group optionally substituted by one or two alkyl or alkoxy groups of up to 3 carbon atoms; $A_2$ is an alkenyl or alkynyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxy of up to 4 carbon atoms.

Suitable esters of the compounds of the formula (II) include the methyl, ethyl, n-propyl, n-butyl, allyl, 2-methylallyl, $CH_2$—C≡CH, methoxymethyl, acetoxymethyl, propionoxymethyl, pivaloyloxymethyl, ethoxycarbonyloxymethyl, methoxycarbonyloxyethyl, ethoxycarbonyloxyethyl, phthalidyl, dimethoxyphthalidyl, benzyl, methoxybenzyl, ethoxybenzyl, nitrobenzyl and chlorobenzyl esters.

Certain favoured groups $A_1$ include methyl, methoxymethyl, acetoxymethyl, acetoxyethyl, phthalidyl, ethoxycarbonyloxymethyl, and $\alpha$-ethoxycarbonyloxyethyl.

Certain favoured groups $A_2$ include the phenyl and 4-methoxyphenyl groups. A particularly favoured moiety $A_3$ is the hydrogen atom.

Esters of the compounds of the formula (II) such as those of compounds of the formulae (III) or (IV) may be presented in the form of their acid addition salts if desired. The acid used to form the salt will most suitably be pharmaceutically acceptable, but non-pharmaceutically acceptable acid addition salts are also envisaged, for example as intermediates in the preparation of the pharmaceutically acceptable salts by ion exchange. Suitable pharmaceutically acceptable acid addition salts include those of inorganic and organic acids, such as hydrochloric, phosphoric, sulphuric, methanesulphonic, toluenesulphonic, citric, malic, acetic, lactic, tartaric, propionic and succinic acid.

Most suitably the acid addition salt is provided as a solid and preferably as a crystalline solid.

Compounds of this invention when in crystalline form may be solvated, for example hydrated.

The present invention provides a pharmaceutical composition which comprises a compound of this invention and a pharmaceutically acceptable carrier.

The compositions of the invention include those in a form adapted for oral, topical or parenteral use and may be used for the treatment of infection in mammals including humans.

Suitable forms of the compositions of this invention include tablets, capsules, creams, syrups, suspensions, solutions, reconstitutable powders and sterile forms suitable for injection or infusion. Such compositions may be made in conventional manner and may contain conventional pharmaceutically acceptable materials such as diluents, binders, colours, flavours, preservatives and disintegrant in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form. The zwitterionic compounds of this invention are particularly suitable for use in such compositions.

The injectable solution of the compound of this invention may be made up in a sterile pyrogen-free liquid such as water or aqueous ethanol.

Compounds of this invention when in highly pure crystalline form tend to have higher aqueous solubilities than those of such previously prepared pure crystalline 9-aminodeoxyclavulanic acid derivatives such as 9-N-benzylaminodeoxyclavulanic acid. This means that the compounds are easier to administer than the previously known compounds. In this circumstance it is often convenient to administer the solution by intra-muscular injection which is less complicated than intravenous administration.

This invention also provides the use of a compound of this invention for preparing injectable aqueous solutions. Such solutions may be prepared by dissolving a sterile compound of this invention in sterile water. Suitably this water is "Water for Injection BP" or the equivalent and may contain electrolytes to render it isotonic.

A particularly suitable injectable aqueous solution of this invention is one which contains not less than 15% w/w of a compound of this invention, favourably not less than 20% w/w of a compound of this invention and preferably not less than 25% w/w of a compound of this invention.

Unit dose compositions comprising a compound of this invention adapted for oral administration form a further suitable composition aspect of this invention. However, orally administrable forms are generally less favoured than injectable forms owing to the relatively poor absorption of the compounds from the gastro-intestinal tract. Despite this orally administrable compositions are of use as a synergistically effective blood level can be expected at high doses and at lower doses such compositions may be used to treat infections localised in the gastro-intestinal tract.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compounds of the invention may be present in the composition as sole therapeutic agent or they may be present together with other therapeutic agents such as a penicillin or cephalosporin. Considerable advantages accrue from the inclusion of a penicillin or cephalosporin since the resulting composition shows enhanced effectiveness (synergy).

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin and other known penicillins including prodrugs therefore such as their in-vivo hydrolysable esters such as the acetoxymethyl, pivaloyloxymethyl, $\alpha$-ethoxycarbonyloxyethyl or phthalidyl esters of ampicillin, benzylpenicillin or amoxycillin, and aldehyde or ketone adducts of penicillins containing a 6-$\alpha$-aminoacetamide side chain (such as hectacillin, metampicillin and analogous derivatives of amoxycillin) or $\alpha$-esters of carbenicillin or ticarcillin such as their phenyl or indanyl $\alpha$-esters.

Suitable cephalosporins for inclusion in the compositions of this invention include cefatrizine, cephaloridine, cephalothin, cefazolin, cephalexin, cephacetrile, cephamandole nafate, cephapirin, cepradine, 4-hydroxycephalexin, cefaparole, cephaloglycin, and other known cephalosporins or prodrugs thereof.

Such compounds are frequently used in the form of a salt or hyrate or the like.

Naturally if the penicillin or cephalosporin present in the composition is not suitable for oral administration then the composition will be adapted for parenteral administration. As previously indicated such injectable or infusable compositions are preferred.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this Invention.

The preferred penicillin for use in the synergistic composition is amoxycillin, for example as its sodium salt or especially as its trihydrate.

Particularly suitable cephalosporins for use in the compositions of this invention include cephaloridine and cefazolin as its pharmaceutically acceptable salt, for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent). Orally administrable compositions containing a compound of the invention will normally contain relatively more synergist than corresponding injectable compositions, for example the ratio in an oral composition may be from about 3:1 to about 1:1 whereas a corresponding injectable composition may contain a ratio of about 1:1 to about 1:3 (compound of the invention: penicillin or cephalosporinl).

The total quantity of a compound of the invention in any unit dosage will normally be between 25 and 1000 mg and will usually be between 50 and 500 mg, for example about 62.5, 100, 125, 150, 200 or 250 mg.

Compositions of this invention may be used for the treatment of infections inter alia, the respiratory tract, the urinary tract and soft tissues in humans and mastitis in cattle.

Normally between 50 and 1000 mg of the compounds of the invention will be administered each day of treatment but more usually between 100 and 750 mg of the compounds of the invention will be administered per day, for example as 1—6 doses, more usually as 2, 3 or 4 doses.

The penicillin or cephalosporin in the synergistic composition of this invention will normally be present at approximately the amount at which it is conventionally used which will usually be expected to be from about 62.5 to 1000 mg per dose, more usually about 125, 250 or 500 mg per dose.

One particularly favoured composition of this invention will contain from 150 to 1000 mg of amoxycillin as the trihydrate or sodium salt and from 25 to 500 mg of a compound of this invention.

The present invention also provides a method of treating bacterial infections in humans or

domestic mammals which comprises the administration of a composition of this invention.

Commonly the infection treated will be due to a strain of *Staphylococcus aureus, Klebsiella aerogenes, Escherichia coli* or *Proteus sp.* The organisms believed to be most readily treated by an antibacterially effected amount of a compound of this invention are strains of *Staphylococcus aureus.* The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The compound of the formula (II) as hereinbefore defined or its ester or pharmaceutically acceptable acid addition salt of such an ester may be prepared by the hydrogenation of a compound of the formula (V):

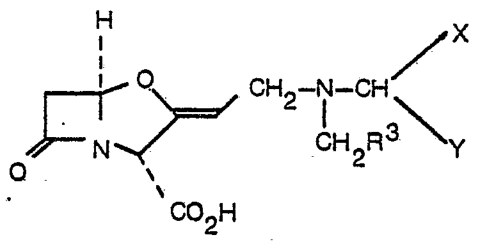

(V)

or ester thereof, wherein X is a group —$CH_2R^1$ or a group that on hydrogenation proves the group $CH_2R^1$ and Y is a group —$CH_2R^2$ or a group that on hydrogenation provides the group $CH_2R^2$, wherein $R^1$ and $R^2$ are as defined with respect to formula (II) above and $R^3$ is a moiety $CR^4{=}CHR^5$ in which $R^4$ is a hydrogen atom or a lower alkyl group and $R^5$ is hydrogen atom, a lower alkyl group or a phenyl group optionally inertly substituted, or $R^3$ is a phenyl group; and optionally thereafter carrying out one or more of the following steps:

i) removing an ester if present
ii) forming an ester
iii) forming a pharmaceutically acceptable acid addition salt of an ester.

When used above the term "lower alkyl" means an alkyl group with 1 to 4 carbon atoms. When used herein suitable inert substituents for use in the phenyl group of $R^5$ include alkyl of 1 to 4 carbon atoms and alkoxy of 1 to 4 carbon atoms.

Suitably X is an alkyl group of 1 to 5 carbon atoms, an alkenyl group of 2 to 5 carbon atoms or an alkynyl group of 2 to 5 carbon atoms. Suitably Y is an alkyl group of 1 to 5 carbon atoms, an alkenyl group of 2 to 5 carbon atoms or an alkynyl group of 2 to 5 carbon atoms. Alternatively X and Y together may represent an alkylene group of 2 to 8 carbon atoms or an alkenylene group 2 to 8 carbon atoms.

It is preferred that X has the value $CH_2R^1$, and that Y has the value $CH_2R^2$.

Suitably $R^3$ is a phenyl group.

Particularly suitable groups $CR^4{=}CHR^5$ include $CH{=}CHCH_3$, $CH{=}CHC_6H_5$, $C(CH_3){=}CH_2$, $C(nC_3H_7){=}CH_2$, $C(CH_3){=}CHCH_3$, $C(CH_3){=}C(CH_3)_2$, $C(CH_3){=}CHC_2H_5$, $C(CH_3){=}CHC_6H_5$, and $C(C_2H_5){=}CH_2$.

Favoured groups $CR^4{=}CHR^5$ are $CH{=}CHCH_3$, $CH{=}CHC_6H_5$, $C(CH_3){=}CH_2$ and $C(CH_3){=}CHC_6H_5$.

Preferred groups $R^3$ are $C_6H_5$, $C(CH_3){=}CH_2$ and $C(CH_3){=}CHC_6H_5$.

The hydrogenation is normally carried out in the presence of a transition metal catalyst.

The catalyst we have preferred to use is palladium, for example in the form of palladium on carbon (charcoal), palladium on barium sulphate, palladium on calcium carbonate and palladium black.

A favoured catalyst is palladium on carbon (sometimes referred to as palladium on charcoal); for example 5%, 10%, 20% or 30% palladium on carbon. The high palladium content catalyst are paricularly apt as smaller total weights of catalyst can be employed thereby avoiding possible problems associated with adsorption of product onto the carbon.

A low, medium or high pressure of hydrogen may be used in this reaction, for example from 1 to 6 atmospheres.

In general if the catalyst used contains a lower percentage of palladium (for example 5% or 10% palladium) then better yields of the desired product are obtained using a pressure of about 3 to 5 atmospheres of hydrogen, for example about 4 atmospheres of hydrogen. In general if the catalyst used contains a higher percentage of palladium (for example 20% or 30% palladium) then acceptable yields of the desired product may also be obtained at low and medium pressures of hydrogen, for example about 1 to 2 atmospheres of hydrogen. We have found it convenient to use an atmospheric or slightly superatmospheric pressure of hydrogen in conjunction with higher palladium content catalysts.

The reaction is normally carried out at a non-extreme temperature, for example from 0°C to 30°C

and more usually from 12°C to 25°C. It is generally convenient to carry out the reaction at ambient temperature.

Suitable solvents for carrying out the hydrogenation include ethanol, n-propanol, isopropanol, tetrahydrofuran, dioxane, ethyl acetate or mixtures of such solvents or such solvents in the presence of water. A favoured solvent is aqueous tetrahydrofuran. A further favoured solvent is a mixture of isopropanol, tetrahydrofuran and water.

We have preferred to carry out the hydrogenation reaction on a hydrogenolysable ester of a compound of the formula (V) so that a compound of the formula (II) per se is formed by the hydrogenation. Such a hydrogenation reaction proceeds at least in part via the formation of a compound of the formula (V). Favoured hydrogenolysable esters include benzyl and substituted benzyl esters such as methoxybenzyl, nitrobenzyl (for example the p-nitrobenzyl ester), chlorobenzyl and bromobenzyl esters. A particularly suitable hydrogenolysable ester is the benzyl ester. A further particularly suitable hydrogenolysable ester is the p-methoxybenzyl ester. Further favoured hydrogenolysable ester groups include those groups $CH_2CR^4{=}CHR^5$ that have been specified hereinbefore as being favoured for removal from a nitrogen atom by hydrogenolysis.

If the hydrogenation is performed on a non-hydrogenolysable ester of the compound of the formula (V) then naturally an ester of the compound of the formula (II) results.

The product may generally be isolated from the reaction mixture by filtering off the solids (the catalyst, which should be well washed to remove the product) and then evaporating the solvent, preferably under low pressure, to yield the initial product. Further purification may be effected by such conventional methods as chromatography over cellulose or other mild stationary phase eluting with a $C_{1-4}$ alkanol optionally in the presence of water and optionally in the presence of ethyl acetate. Evaporation of the combined active fraction (identified by aqueous potassium permanganate spray on tlc) then yields the desired compound in pure form. The desired product is normally obtained in crystalline form (unless it is an unsalted ester). Trituration under ethanol, isopropanol or similar $C_{1-4}$ alkanol or other conventional solvent such as a ketone, ether or ester solvent (for example of up to 6 carbon atoms and more suitably of up to 4 carbon atoms) may also be used to aid crystallisation. Recrystallisation from ethanol may also be employed. The solvent used in such processes may advantageously be moist. The preceding work up procedures successfully separate the desired compound from other products of the hydrogenation such as 9-N,N-dialkylaminodeoxyclavulanates.

Zwitterionic compounds of the formula (IIa) may be obtained from high yielding reactions by the addition of a $C_{1-4}$ alkanol such as cold ethanol to the initial product.

If the initial product contains impurities it may be advantageous to wash the initial product by dissolving in a water immiscible organic solvent and extracting into water. Evaporation of the aqueous phase, preferably under a good vacuum, then yields a purer product which may be further purified if desired as previously described.

Certain unsalted esters of the compounds of the formula (II) are low melting so that it is often more convenient for handling to convert them into solid acid addition salts, for example by reaction with one equivalent of an acid. Alternatively the non-hydrogenolysable ester of the compound of the formula (V) may be hydrogenated in the presence of one equivalent of an acid, that is they may be hydrogenated in the form of their acid addition salt.

In an alternative aspect of this invention there is provided a process for the preparation of a compound of the formula (II) as hereinbefore defined or an ester or acid addition salt of such an ester which process comprises the reaction of a compound of the formula (VI), or a derivative thereof which allows alkylation to take place:

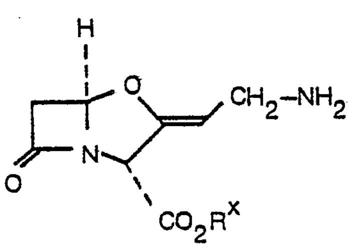

(VI)

wherein $R^x$ is a hydrogen atom or a ester group, with a compound of the formula (VII):

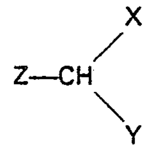

(VII)

wherein X and Y are as defined in relation to formula (V) and Z is a readily displaceable group; and optionally thereafter:

i) removing an ester if present,
ii) forming an ester,
iii) forming a pharmaceutically acceptable acid addition salt of an ester,
iv) converting any groups X and Y to groups $CH_2R^1$and $CH_2R^2$.

Preferably the readily displaceable group is bromide, iodide or a sulphonate ester such as a tosylate.

Normally the reaction is carried out in the presence of a non-nucleophilic base, for example diazabicyclononene or diazabicycloundecane.

If $R^x$ is a hydrogen atom then reaction will occur also on the free acid function to provide an alkyl ester. There is a tendency for di-alkylation on the nitrogen atom to occur thus forming a tertiary amine. However, this possibility may be avoided or minimised by careful selection of reagent quantities and/or reaction conditions.

If the groups X and Y are moieties that can be converted to groups $CH_2R^1$ and $CH_2R^2$ respectively then this can be performed using standard methods of hydrogenolysis as hereinbefore described.

If it is desired to remove the ester group then this may be done by standard chemical methods appropriate to the nature of the ester, such standard chemical methods include hydrogenation, acid and base hydrolysis and enzymatic hydrolysis.

The compounds of the formula (VI) can be prepared by the methods of Belgian Patent Nos. 866496 and 855375 Derivatives of the formula (VI) formed on the 9-amino function that allow alkylation to take place on the 9-amino function are those well-known in the art, for example a silyl derivative.

A pharmaceutically acceptable ester of a compound of the formula (II) may be prepared by reaction of the compound of the formula (II) with an esterifying agent.

The zwitterionic compound of the formula (II) may be dissolved or suspended in a solvent such as dimethylformamide, hexamethylphosphoramide, dichloromethane, ethyl acetate or other non-esterifiable solvents and therein esterified. Suitable temperatures for such a reaction range from about 0° to about 25°C. Suitable esterifying reagents include reactive halides and their equivalents and alkyl oxonium salts.

When a reagent such as a reactive iodide, chloride, bromide, tosylate, mesylate or the equivalent is used, the resulting salt is generally suitable for use in a composition of this invention. Alternatively, the salt may be converted to a free base or alternative salt. When an alkyl oxonium salt is used, it is preferred to convert the resulting tetrafluoroborate to the free base or alternative salt. The various afore-mentioned salts may be converted to the free base by neutralisation, for example by contacting a solution of the salt in water with an organic phase, neutralising the salt by adding a base and extracting the liberated amine into the organic phase. This amine may thereafter be re-salted by reacting with an appropriate acid, for example in a dry organic solvent. It is generally preferred to use not more than one equivalent of acid for this process. Alternatively, the originally formed salt may be converted into the alternative salt using an ion exchange material for example, by passing an aqueous solution of one salt through a bed of an anion exchange resin in the form of the desired salt such as the chloride form.

The salts may normally be obtained in solid form by dissolving in a fairly polar organic solvent (such as ethanol or tetrahydrofuran) and then precipitating using a less-polar solvent such as diethyl ether or cyclohexane.

The salts of the esters of the compounds of the formula (II) may normally be obtained in crystalline form by conventional methods such as trituration under (or crystallisation or recrystallisation from) a suitable organic solvent such as ether, acetone, acetonitrile or tetrahydrofuran.

Esters of the compound of the formula (II) may also be prepared by reaction of an acid addition salt of the compound of the formula (II) with an alcohol in the presence of a condensation promoting agent.

Suitable condensation promoting agents for use in this process include carbodiimides such as dicyclohexylcarboxiimide and the chemical equivalents thereof.

The acid addition salt may be formed in situ or may be preformed. The acid employed will normally be a strong acid such as a methane sulphonic acid, p-toluene sulphonic acid or trifluoroacetic acid.

The reaction is normally carried out in an inert organic solvent. When the ester being formed is that of a liquid alcohol it is convenient to use that alcohol as the solvent or as part of the solvent system. The esterification is generally performed at a non-extreme temperature such as 0° to 35°C, for example from about 10° to 25°C. Conveniently the reaction may be performed at ambient temperature.

Since the compound of the formula (V) and its salts and esters are of use as intermediates they form part of this invention. Suitably the compounds of the formula (V) are in the form of an ester of a type hereinbefore described. Suitably the compounds of the formula (V) are in the form of a salt such as in alkali metal salt, for example the lithium salt.

The intermediates of the formula (V) and its salts and esters may be prepared by the methods of

British Application No. 41887/75, U.S. Serial No. 731928, Belgian Patent No. 847044 or West German Offenlegungsschrift 2646003.

The present invention also provides a process for the preparation of an ester or acid addition salt thereof of a compound of the formula (II) which process comprises the hydrogenation of a compound of the formula (VIII):

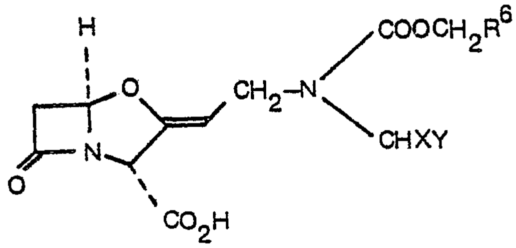

(VIII)

or an ester thereof wherein X and Y are as defined in relation to formula (V), and $R^6$ is a moiety of the sub-formula a)

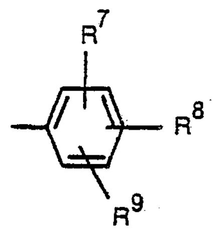

a)

wherein $R^7$ is a hydrogen, fluorine, chlorine or bromine atom or an alkyl group of 1—3 carbon atoms, an alkoxy group of 1—3 carbon atoms, an acyloxy group of 1—3 carbon atoms, a hydroxyl group, an alkoxycarbonyl group containing 1—3 carbon atoms in the alkoxy part, or a group —$N(R^{10})CO.R^{11}$, —$N(R^{10})SO_2R^{11}$ or —CO—$NR^{10}R^{11}$ where $R^{10}$ is a hydrogen atom or an alkyl group of 1—3 carbon atoms or a phenyl or benzyl group and $R^{11}$ is an alkyl group of 1—3 carbon atoms or a phenyl or benzyl group; $R^8$ is a hydrogen, fluorine or chlorine atom or an alkyl group of 1—3 carbon atoms, an alkoxyl group of 1—3 carbon atoms or an acyloxy group of 1—3 carbon atoms; and $R^9$ is a hydrogen, fluorine or chlorine atom or an alkyl group of 1—3 carbon atoms or an alkoxyl group of 1—3 carbon atoms; and optionally thereafter

i) removing an ester if present,
ii) forming an ester,
iii) forming a pharmaceutically acceptable addition salt of an ester.

Most suitably $R^6$ is a phenyl group.

Particularly suitable esters of the compound of the formula (VIII) include $C_{1-4}$ alkyl esters especially the methyl and ethyl esters.

The hydrogenation can be performed in the same general manager as hereinbefore described in relation to the hydrogenation of a compound of the formula (V).

An ester of the compound of the formula (VIII) may be formed from the free acid or salt in conventional manner.

The compound of the formula (VIII) or ester thereof can be prepared by the reaction of a compound of the formula (IX) or an ester thereof;

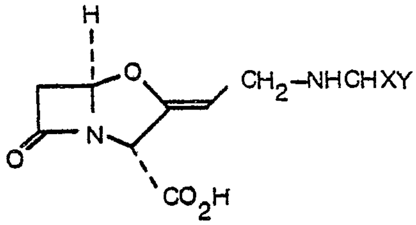

(IX)

wherein X and Y are as defined in relation to formula (V), with a compound of the formula (X):

$$T—CO—O—R^6 \qquad (X)$$

wherein $R^6$ is as defined in relation to formula (VIII) and T is a readily displaceable group.

Favoured groups T include the chlorine and bromine atoms, and sulphonate and carboxylate esters.

A preferred compound of the formula (X) is benzyl chloroformate.

The reaction may be performed under conventional acylation conditions, for example in non-acylatable organic solvents such as acetone in the presence of an acid acceptor such as lithium bicarbonate, sodium carbonate or potassium carbonate at a non-extreme temperature such as −10°C to 30°C.

Therefore in a broad aspect there is provided a process for the preparation of a compound of the formula (II) or its ester or pharmaceutically acceptable acid addition salt of such an ester, which process comprises the hydrogenation of a compound of the formula (V) or ester thereof, or the reaction of a compound of the formula (VII) with a compound of the formula (V) or ester thereof, and optionally therafter:

i) acylating the compound of the formula (II) or its ester with a compound of the formula (IX),
ii) hydrogenating the compound of the formula (VIII),
iii) removing an ester if present,
iv) forming an ester,
iv) forming a pharmaceutically acceptable acid addition salt of an ester.

The following examples serve to illustrate the invention.

### Example 1

a) Benzyl 9-N-isopropyl-N-(2′-methyl-3′-phenylallyl)aminodeoxyclavulanate

Benzyl dichloroacetylclavulanate (2.0 g; 5mM) in dry dimethylformamide (30 $cm^3$) was treated with 1.9 equivalents of *N*-isopropyl-N-(2-methyl-3-phenyl allyl) amine at −15° and allowed to warm up to room temperature over 4 hours. The mixture was poured into ethyl acetate (200 $cm^3$) and was washed with water (5 x 100 $cm^3$) and saturated brine, dried (anhydrous magnesium sulphate) and evaporated to an oil. This oil was chromatographed on silica eluting with ethyl acetate-cyclohexane, 1:2. Fractions were collected containing the title compound Rf ($SiO_2$/ethylacetate:cyclohexane; 1:1) = 0.82. Combined fractions were evaporated to give an oil, 80 mg $\nu_{max}$ (film) 1805, 1750, 750, 700 $cm^{-1}$. The proton magnetic resonance spectrum was consistent with the desired product.

b) 9-*N*-Isopropylaminodeoxyclavulanic acid

Benzyl 9-N-isopropyl-*N*-(2′-methyl-3′-phenylallyl)aminodeoxyclavulanate (76 mg) in ethanol (20 $cm^3$) was hydrogenolysed at atmospheric pressure in the presence of 25 mg palladium on carbon (10% Pd; prehydrogenated for 10 minutes) for 40 minutes. The catalyst was filtered off and washed with aqueous ethanol (10 $cm^3$) and the filtrate evaporated to an oil. This oil was chromatographed on silica eluting with ethylacetate:ethanol:water; 5:2:2. Fractions were collected containing the title compound, Rf ($SiO_2$/ethylacetate:ethanol:water; 5:2:5) = 0.23. Combined fractions were evaporated, acetonitrile added and again evaporated to give a white solid, yield = 10 mg. $\nu_{max}$ (Nujol) (3100—2200) very broad, 1805, 1700, 1622, 1575 $cm^{-1}$. $\delta$ ($D_2O$) 1.18 (6H, d, *J* 6 Hz), 2.97 (1H, d, *J* 17 Hz), 3.0—3.5 (1H, m), 3.47 (1H, dd, *J* 17 and 3 Hz), 3.58 (2H, d, *J* 7.5 Hz), (1H, broad t, *J* 7.5 Hz), 4.85 (1H, broad s), 5.62 (1H, d, *J* 3 Hz).

### Example 2

a) Benzyl 9-N-cyclopentyl-N-(2′-methyl-3′-phenylallyl)aminodeoxyclavulanate

Benzyl dichloroacetylclavulanate (5 g; 12.5 mM) in dry dimethylformamide (60 $cm^3$) at −15° was treated with 1.9 equivalents of *N*-cyclopentyl-N-(2-methyl-3-phenylallyl)amine, dropwise with stirring over 10 minutes, then stirred at −5° for 1½ hours and between −5° and +10° over 2 hours. The mixture was poured into ethylacetate (250 $cm^3$) and washed with water (5 x 200 $cm^3$), saturated brine 5 x 200 $cm^3$), dried (anhydrous magnesium sulphate) and evaporated in the presence of toluene to small volume. This crude product was chromatographed on silica eluting with ethylacetate-cyclohexane (1:3). Fractions were collecting containing the title compound, Rf ($SiO_2$/ethylacetate-cyclohexane; 1:3) = 0.5 (detection by aqueous potassium permanganate spray), combined fractions were evaporated to an oil, yield = 1.05 g (17%), $\nu_{max}$ (film) 1803, 1750, 1698, 1302, 1172, 1010, 742, 698 $cm^{-1}$, $\delta$ ($CDCl_3$) 1.20—2.00 (8H, broad m), 1.84 (3H, s) 2.70—3.15 (2H, broad m), 2.98 (2H, s), 3.26 (2H, d, *J* 7 Hz), 3.36 (1H, dd, *J* 17 and 3 Hz), 4.77 (1H, broad t, *J* 7 Hz), 5.05 (1H, s), 5.15 (2H, s), 5.57 (1H, d, *J* 3 Hz), 6.35 (1H, broad s), 7.24 and 7.32 (10H, 2 x broad s).

b) 9-N-Cyclopentylaminodeoxyclavulanic acid

Benzyl 9-*N*-cyclopentyl-*N*-(2-methyl-3-phenylallyl)aminodeoxyclavulanate (0.78 g; 1.6 mM) in ethanol (30 $cm^3$) was hydrogenolysed at atmospheric pressure in the presence of 10% palladium on carbon (250 mg, which had been prehydrogenated for 10 minutes) for 1 hour and 20 minutes when water (1 $cm^3$) was added and hydrogenolysis continued for ¾ hour. The catalyst was filtered off and washed with aqueous ethanol (50 $cm^3$), the filtrate was evaporated and acetonitrile added. The solution was cooled (0°) and the resulting crystals filtered off, washed with cold acetonitrile and dried to afford

the title compound as a colourless crystalline solid; yield = 185 mg (43%), Rf ($SiO_2$/ethylacetate-ethanol-water; 5:2:2) = 0.48 $\nu_{max}$ (Nujol) (3150—2100 broadl), 1808, 1700, 1630, 1572, 1290, 1190, 1120, 1047, 1015, 1005, 920, 892, 820, 750 $cm^{-1}$, $\nu_{max}$ (KBr) (3120—2200 multiple and broad), 1800, 1696, 1618, 1568, 1477, 1390, 1303, 1286, 1185, 1157, 1120, 1045, 1013, 1003, 915, 892, 822, 787, 752 $cm^{-1}$. $\delta$ ($D_2O$) 1.35—2.25 (8H, broad m), 3.10 (1H, d, *J* 17 Hz), 3.58 (1H, dd, *J* 17 and 3 Hz), 3.30—3.85 (1H, broad m), 3.71, (2H, d, *J* 8 Hz), 4.79 (1H, broad t, *J* 8 Hz), 4.98 (1H, d, *J* 3 Hz).

Example 3

a) Benzyl 9-N-cyclohexyl-N-(2′-methyl-3′-phenylallyl)aminodeoxyclavulanate

Benzyl dichloroacetylclavulanate (4 g; 10mM) in dry dimethylformamide (40 $cm^3$) at −15° was treated with 1.9 equivalents of *N*-cyclohexyl-N-(2-methyl-3-phenylallyl)amine, dropwise over 15 minutes with stirring. The mixture was stirred for 2 hours between −15° and +10°, then poured into ethylacetate (200 $cm^3$) and washed with water (4 x 100 $cm^3$) and saturated brine (4 x 100 $cm^3$), dried (anhydrous magnesium sulphate) and evaporated in the presence of toluene to low volume, this crude product was chromatographed on silica eluting with ethylacetate-cyclohexane; 1:3. Fractions were collected containing the title compound, Rf ($SiO_2$/ethylacetate-cyclohexane; 1:2) = 0.7 (detection by aqueous potassium permanganate spray). Combined fractions were evaporated to an oil, yield = 218 mg, $\nu_{max}$ (film) 1805, 1750, 1700, 1450, 130S, 1172, 1012, 890, 745, 700 $cm^{-1}$. $\delta$ ($CDCl_3$) 0.70—2.00 (10H, broad m), 1.82 (3H, s), 2.25—2.65 (1H, broad m), 2.93 (1H, d, *J* 17 Hz), 3.05 (2H, s), 3.20 (2H, d, *J* 7 Hz), 3.40 (1H, dd, *J* 17 and 3 Hz), 4.72 (1H, broad t, *J* 7 Hz), 5.05 (1H, s), 5.15 (2H, s), 5.60 (1H, d, *J* 3 Hz), 6.38 (1H, broad s), 7.25 and 7.32 (10H, 2 x s).

b) 9-*N*-Cyclohexylaminodeoxyclavulanic acid

Benzyl 9-*N*-cyclohexyl-*N*-(2-methyl-3-phenylallyl)aminodeoxyclavulanate (310 mg; 0.62 mM) in ethanol (25 $cm^3$) was hydrogenolysed at atmospheric pressure in the presence of 10% palladium on carbon (100 mg; which had been prehydrogenated for 10 minutes) for 1½ hours. The catalyst was filtered off and washed with ethanol (30 $cm^3$), then with aqueous ethanol (100 $cm^3$), this aqueous washing was collected separately and evaporated to a white solid. Methanol was added (5 $cm^3$) and the crystals filtered off cold (0°) and dried to give 2.7 mg of the title compound. The filtrate was evaporated and cold acetonitrile added, crystals were filtered off and washed with cold acetonitrile, drying afforded a further 12 mg of the title compound. The ethanolic washings from the catalyst and the reaction filtrate were evaporated to an oil, acetonitrile was added and the solution cooled. Crystals slowly formed, these were filtered off and washed with cold acetonitrile, drying afforded 30 mg of the title compound. Total yield = 44 mg (26%) Rf ($SiO_2$/ethylacetate-ethanol-water; 5:2:2) = 0.60.$\nu_{max}$ (Nujol) 1802, 1695, 1615, 1582, 1300, 1185, 1112, 1065, 1045, 1020, 1002, 930, 895, 815, 755 $cm^{-1}$; $\nu_{max}$ (KBr) (3700—2200 broad multiple peaks), 1802, 1698, 1615, 1580, 1395, 1300, 1194, 1120, 1070, 1046, 1022, 935, 898, 760 $cm^{-1}$. $\delta$ ($D_2O$) 0.75—2.25 (10H, broad m), 2.75—3.25 (1H, broad m), 3.03 (1H, d, *J* 17 Hz), 3.55 (1H, dd, *J* 17 and 3 Hz), 3.67 (2H, d, *J* 8 Hz), 4.73 (1H, t, *J* 8 Hz), 4.94 (1H, s), 5.72 (1H, d, *J* 3 Hz).

Example 4

a) Benzyl 9-*N*-cyclopropyl-N-(2′-methyl-3′-phenylallyl)aminodeoxyclavulanate

Benzyl 9-O-dichloroacetylclavulanate (6.98 g; 17.5 mmol) in 'dry' dimethylformamide (70 ml) was cooled with stirring to −20°C. N-Cyclopropyl-N-(2′-methyl-3′-phenylallyl)amine (6.2 g; 33 mmol) in 'dry' dimethylformamide (60 ml) was added dropwise over a period of 20 minutes. The reaction mixture was then stirred between −20°C and +10° for a period of 2 hours.

The solution was then poured into an ethyl acetate/water mixture and shaken. The two layers were separated and the aqueous phase extracted with ethyl acetate. The combined organic layers were then washed several times with a saturated solution of sodium chloride, dried ($MgSO_4$) and evaporated to an oil. Column chromatography afforded the title compound as a crystalline solid in 21% yield, $\nu_{max}$ ($CHCl_3$): 1795, 1742 and 1690 $cm^{-1}$; $\delta$ ($CDCl_3$) 0.34 (4H, m, $CH_2$—CH—$CH_2$), 1.73 (1H, m, $CH_2$—*CH*—$CH_2$), 1.80 (3H, s, C—$CH_3$), 2.91 (1H, d, *J* 17 Hz, 6β-*CH*), 3.12 (2H, s, N—$CH_2$), 3.27 (2H, d, *J* 7 Hz, 9-$CH_2$), 3.39 (1H, dd, 6α-*CH*, partially obscured by 9-$CH_2$), 4.81 (1H, br.t, *J* 7 Hz, 8-*CH*), 5.07 (1H, s, 3-*CH*), 5.20 (2H, s, $CO_2CH_2$), 5.60 (1H, d, *J* 3 Hz, 5-*CH*), 6.31 (1H, s, $CHH_6H_5$), 7.25, 7.30 (10H, 2 x s, aromatic H's).

b) 9-N-Cyclopropylaminodeoxyclavulanic acid

Benzyl 9-N-cyclopropyl-N-(2′-methyl-3′-phenylallyl)aminodeoxyclavulanate (1.4 g; 3 mmol) in ethanol was carefully added to a pre-hydrogenated mixture of 10% palladium on charcoal (700 mg) in ethanol (50 ml). The mixture was then hydrogenated at 1 atmosphere until the uptake of hydrogen ceased (1 hour).

The catalyst was then filtered off through a celite pad and the 'cake' washed well with aqueous ethanol. The filtrate plus washings were then evaporated to give a yellow solid which on trituration with

ethanol afforded the title compound as a white crystalline solid in a 43% yield, $\nu_{max}$ (KBr): 1785, 1695 and 1600 (broad) $cm^{-1}$; $\delta$ ($D_2O$) 0.65—0.95 (4H, m, $CH_2$—CH—$CH_2$); 2.45—2.80 (1H, m, $CH_2$—CH—$CH_2$), 3.04 (1H, d, *J* 17 Hz, 6β-C*H*), 3.54 (1H, dd, *J* 17 and 3 Hz, 6α-C*H*), 3.74 (2H, d, *J* 7 Hz, 9-$CH_2$), 4.78 (1H, broad t, *J* 7 Hz, 8-C*H*), 4.93 (1H, s, 3-C*H*) and 5.71 (1H, d, *J* 3 Hz, 5-C*H*).

Example 5

Composition

a) A solution for injection may be prepared by dissolving 100 mg of sterile 9-*N*-isopropylaminodeoxyclavulanic acid in 1 ml of sterile water.

b) A solution for injection may be prepared by dissolving 50 mg of sterile 9-N-isopropylaminodeoxyclavulanic acid and 250 mg of sterile sodium amoxycillin in 1 ml of sterile water.

c) A solution for injection may be prepared by dissolving 125 mg of sterile 9-N-isopropylaminodeoxyclavulanic acid and 125 mg of sterile cephaloridine in 1.5 ml of sterile water.

*In vitro data*

Demonstration of Effectiveness

The following results were obtained in a standard MIC test using ampicillin, 9-N-isopropylaminodeoxyclavulanic acid, 9-N-cyclopentylaminodeoxyclavulanic acid, and 9-N cydohexylaminodeoxyclavulanic acid.

Synergistic and antibacterial properties of 9-N-isopropylaminodeoxyclavulanic acid

| | Staph Russell | Kleb Eto | E.coli JT39 |
|---|---|---|---|
| Ampicillin alone | 125 | 500 | 2000 |
| Ampicillin + clavulanic acid | | | |
| 1.0 | 0.6 | 3.1 | 8.0 |
| 5.0 | 0.02 | 1.5 | 1.0 |
| Ampicillin + 9-N-isopropylamino deoxyclavulanic acid | | | |
| 1.0 | 0.05 | 3.1 | 2.0 |
| 5.0 | Inhibited | 1.5 | 1.0 |
| Clavulanic acid alone | 16 | 31.2 | 31.2 |
| Isopropylamino deoxy-clavulanic acid alone | 8 | 500 | 31.2 |

Synergistic and antibacterial properties of 9-N-cyclohexylaminodeoxyclavulanic acid

| | Staph Russell | Kleb Eto | E.coli JT39 |
|---|---|---|---|
| Ampicillin alone | 1000 | 1000 | 2000 |
| Ampicillin + clavulanic acid | | | |
| 1.0 | 0.6 | 12.5 | 16 |
| 5.0 | 0.08 | 1.6 | 4.0 |
| Ampicillin + 9-N-cyclohexyl-aminodeoxy-clavulanic acid | | | |
| 1.0 | 0.3 | 6.25 | 8.0 |
| 5.0 | Inhibited | 12.5 | 4.0 |
| Clavulanic acid alone | 31.2 | 62.5 | 31.2 |
| 9-N-cyclohexylamino deoxyclavulanic acid | 8.0 | 500 | 62.5 |

Synergistic and antibacterial properties of 9-N-cyclopentylaminodeoxyclavulanic acid

| | Staph Russell | Kleb Eto | E.coli JT39 |
|---|---|---|---|
| Ampicillin alone | 250 | 31.2 | 250 |
| Ampicillin + clavulanic acid | | | |
| 1.0 | 0.3 | 1.5 | 16.0 |
| 5.0 | ≤0.01 | 0.8 | 4.0 |
| Ampicillin + 9-N-cyclopentyl-aminodeoxy-clavulanic acid | | | |
| 1.0 | 0.15 | 3.1 | 8.0 |
| 5.0 | Inhibited | 1.5 | 4.0 |
| Clavulanic acid alone | 31.2 | 62.5 | 16.0 |
| 9-N-cyclopentyl-aminodeoxy-clavulanic acid | 16 | >500 | 31.2 |

In-vivo subcutaneous synergistic activity of the compound of Example 2 with amoxycillin against intraperitoneal E. coli infections in mice.

| | $CD_{50}$ mg/kg E. coli strain | |
|---|---|---|
| | E96 | E124 |
| Amoxycillin alone | >1000 | >1000 |
| Amoxycillin alone + 2.0 mg/kg of compound of example 2 | 7.5 | >100 |

**Claims for the Contracting States: BE CH DE FR GB IT LI LU NL SE**

1. The compound of the formula (II) or an ester or pharmaceutically acceptable acid addition salt thereof:

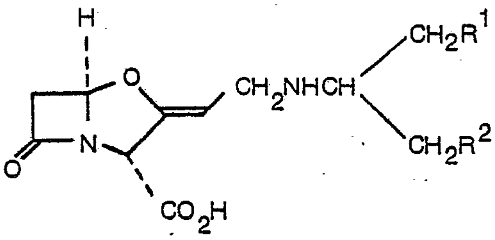

(II)

wherein $R^1$ is a hydrogen atom or a $C_{1-4}$ alkyl group and $R^2$ is a hydrogen atom or a $C_{1-4}$ alkyl group; or $R^1$ and $R^2$ together may represent a bond or a group $-(CH_2)_n-$ where n is an integer from 1 to 6.

2. A compound as claimed in claim 1 selected from 9-N-isopropylaminodeoxyclavulanic acid,
9-N-cyclopropylaminodeoxyclavulanic acid,
9-N-cyclobutylaminodeoxyclavulanic acid,
9-N-cyclopentylaminodeoxyclavulanic acid, and
9-N-cyclohexylaminodeoxyclavulanic acid.

3. A compound as claimed in either claim 1 or claim 2 when in crystalline form.

4. A compound as claimed in claim 1 when in the form of a free acid.

5. An ester as claimed in claim 1 wherein the ester is of the sub-formula $A_1$ or $CHA_2A_3$:

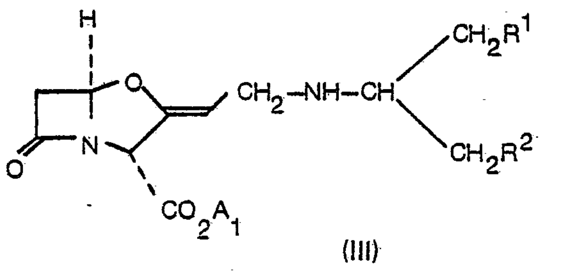

(III)

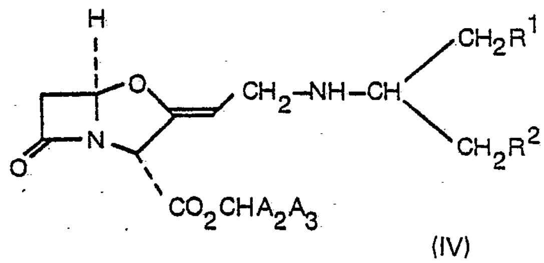

(IV)

wherein $A_1$ is $C_{1-6}$ alkyl optionally substituted by alkoxy, aryloxy, alkoxycarbonyloxy, acyloxy, aralkanoyl, alkanoyl or benzoyl of up to 8 carbon atoms, any phenyl groups in such substituents being themselves optionally substituted by a fluorine, bromine, chlorine, nitro, $C_{1-4}$ alkyl or $C_{1-4}$ alkoxy; or is a phthalidyl group optionally substituted by one or two alkyl or alkoxy groups of up to 3 carbon atoms; $A_2$ is an alkenyl or akynyl group of up to 5 carbon atoms or is a phenyl group optionally substituted by a fluorine, chlorine, bromine, nitro or alkyl or alkoxy of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

6. An ester as claimed in either claim 1 or claim 5 in the form of an acid addition salt.

7. A pharmaceutical composition which comprises a compound as claimed in any of claims 1 to 6 and a pharmaceutically acceptable carrier.

8. A composition as claimed in claim 7 which also contains a penicillin or cephalosporin.

9. A process for the preparation of a compound of the formula (II) or its ester or pharmaceutically acceptable acid addition salt thereof which process comprises the hydrogenation of a compound of the formula (V):

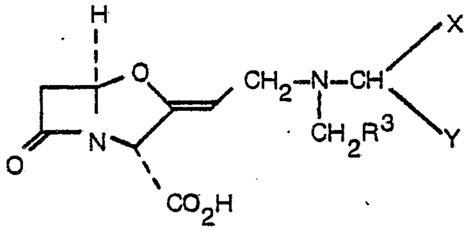

(V)

or ester thereof, wherein X is a group $-CH_2R^1$ or a group that on hydrogenation proves the group $CH_2R^1$ and Y is a group $-CH_2R^2$ or a group that on hydrogenation provides the group $CH_2R^2$, wherein $R^1$ and $R^2$ are as defined in claim 1 and $R^3$ is a moiety $CR^4{=}CHR^5$ in which $R^4$ is a hydrogen atom or a $C_{1-4}$ alkyl group and $R^5$ is hydrogen atom, a $C_{1-4}$ alkyl group or a phenyl group optionally inertly substituted, or $R^3$ is a phenyl group; and optionally thereafter carrying out one or more of the following steps:

i) removing an ester if present
ii) forming an ester
iii) forming a pharmaceutically acceptable acid addition salt of an ester.

10. A process for the preparation of a compound of the formula (II) or its ester or acid addition salt thereof which process comprises the reaction of a compound of the formula (VI), or a derivative thereof which allows alkylation to take place:

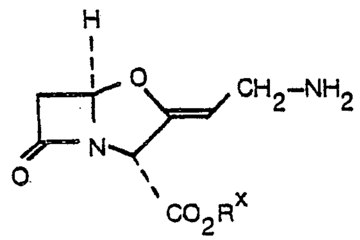

(VI)

wherein $R^x$ is a hydrogen atom or a ester group, with a compound of the formula (VII):

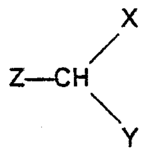

(VII)

wherein X and Y are as defined in relation to formula (V) and Z is a readily displaceable group; and optionally thereafter:

i) removing an ester if present,
ii) forming an ester,
iii) forming a pharmaceutically acceptable acid addition salt of an ester,
iv) converting any groups X and Y to groups $CH_2R^1$ and $CH_2R^2$.

11. A process for the preparation of a compound of the formula (II) or its ester or acid addition salt thereof which process comprises the hydrogenation of a compound of the formula (VIII):

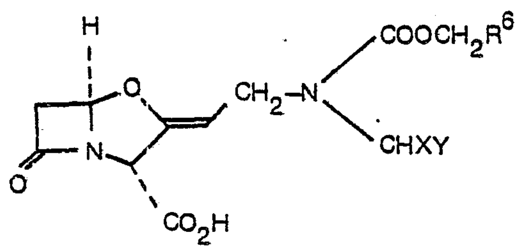

(VIII)

or an ester thereof wherein X and Y are as defined in relation to formula (V), and $R^6$ is a moiety of the sub-formula a)

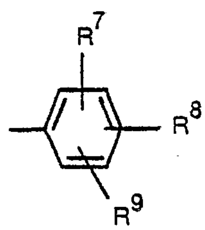

a)

wherein $R^7$ is a hydrogen, fluorine, chlorine or bromine atom or a alkyl group of 1—3 carbon atoms, an alkoxyl group of 1—3 carbon atoms, an acyloxyl group of 1—3 carbon atoms, a hydroxyl group, an alkoxycarbonyl group containing 1—3 carbon atoms in the alkoxy part, or a group $—N(R^{10})CO.R^{11}$, $—N(R^{10})SO_2R^{11}$ or $—CO—NR^{10}R^{11}$ where $R^{10}$ is a hydrogen atom or an alkyl group of 1—3 carbon atoms or a phenyl or benzyl group and $R^{11}$ is an alkyl group of 1—3 carbon atoms or a phenyl or benzyl group; $R^8$ is a hydrogen, fluorine or chlorine atom or an alkyl group of 1—3 carbon atoms, an alkoxyl group of 1—3 carbon atoms or an acyloxyl group of 1—3 carbon atoms; and $R^9$ is a hydrogen fluorine or chlorine atom or an alkyl group of 1—3 carbon atoms or an alkoxyl group of 1—3 carbon atoms; and optionally thereafter

i) removing an ester if present,
ii) forming an ester,
iii) forming a pharmaceutically acceptable addition salt of an ester.

12. A process for the preparation of a compound of the formula (II) or its ester or acid addition salt thereof which process comprises the hydrogenation of a compound of the formula (V) or ester thereof, or the reaction of a compound of the formula (VII) with a compound of the formula (VI) or ester thereof, optionally thereafter:

i) acylating the compound of the formula (II) or its ester with a compound of the formula (IX),
ii) hydrogenating the compound of the formula (VIII),
iii) removing an ester if present,
iv) forming an ester,
v) forming a pharmaceutically acceptable acid addition salt of an ester.

**Claims for the contracting state: AT**

1. A process for the preparation of a compound of the formula (II): or an ester or pharmaceutically acceptable acid addition salt thereof

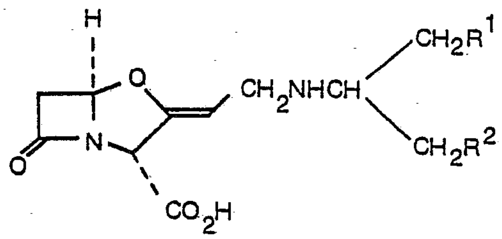

(II)

wherein $R^1$ is a hydrogen atom or a $C_{1-4}$ alkyl group and $R^2$ is a hydrogen atom or a $C_{1-4}$ alkyl group; or $R^1$ and $R^2$ together may a represent a bond or a group $-(CH_2)_n-$ where n is an integer of a compound of the formula (V):

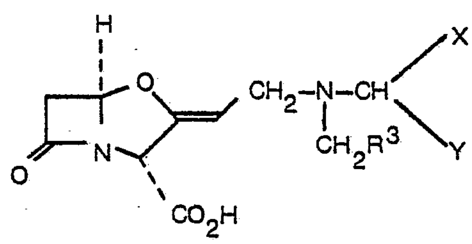

(V)

or ester thereof, wherein X is a group $-CH_2R^1$ or a group that on hydrogenation proves the group $CH_2R^1$ and Y is a group $-CH_2R^2$ or a group that on hydrogenation provides the group $CH_2R^2$, wherein $R^1$ and $R^2$ are as defined with respect to formula (I) above and $R^3$ is a moiety $CR^4{=}CHR^5$ in which $R^4$ is a hydrogen atom or a $C_{1-4}$ alkyl group and $R^5$ is hydrogen atom, a $C_{1-4}$ alkyl group or a phenyl group optionally inertly substituted, or $R^3$ is a phenyl group; and optionally inertly substituted, or $R^3$ is a phenyl group; and optionally thereafter carrying out one or more of the following steps:

i) removing an ester if present
ii) forming an ester
iii) forming a pharmaceutically acceptable acid addition salt of an ester.

2. A process for the preparation of a compound of the formula (II) as defined in claim 1 or its ester or acid addition salt thereof which process comprises the reaction of a compound of the formula (VI), or a derivative thereof which allows alkylation to take place:

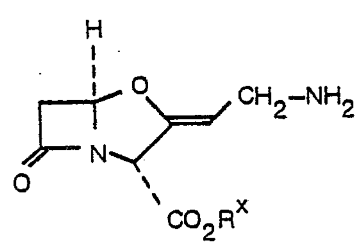

(VI)

wherein $R^x$ is a hydrogen atom or a ester group, with a compound of the formula (VII):

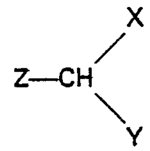

(VII)

wherein X and Y are as defined in relation to formula (V) and Z is a readily displaceable group; and optionally thereafter:

i) removing an ester if present,
ii) forming an ester,
iii) forming a pharmaceutically acceptable acid addition salt of an ester,
iv) converting any groups X and Y to groups $CH_2R^1$ and $CH_2R^2$.

3. A process for the preparation of a compound of the formula (II) as defined in claim 1 or its ester or acid addition salt thereof which process comprises the hydrogenation of a compound of the formula (VIII):

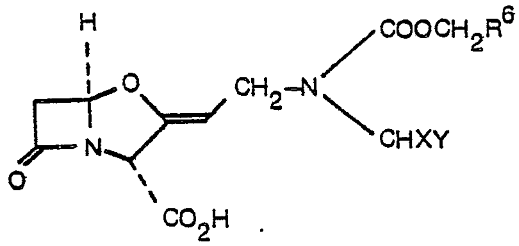

(VIII)

or an ester thereof wherein X and Y are as defined in relation to formula (V), and $R^6$ is a moiety of the sub-formula a)

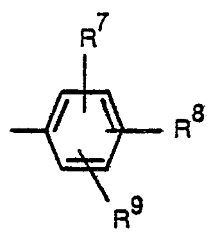

a)

wherein $R^7$ is a hydrogen, fluorine, chlorine or bromine atom or an alkyl group of 1—3 carbon atoms, an alkoxyl group of 1—3 carbon atoms, an acyloxyl group of 1—3 carbon atoms, a hydroxyl group, an alkoxycarbonyl group containing 1—3 carbon atoms in the alkoxy part, or a group $—N(R^{10})CO.R^{11}$, $—N(R^{10})SO_2R^{11}$ or $—CO—NR^{10}R^{11}$ where $R^{10}$ is a hydrogen atom or an alkyl group of 1—3 carbon atoms or a phenyl or benzyl group and $R^{11}$ is an alkyl group of 1—3 carbon atoms or a phenyl or benzyl group; $R^8$ is a hydrogen, fluorine or chlorine atom or an alkyl group of 1—3 carbon atoms, an alkoxyl group of 1—3 carbon atoms or an acyloxyl group of 1—3 carbon atoms; and $R^9$ is a hydrogen fluorine or chlorine atom or an alkyl group of 1—3 carbon atoms or an alkoxyl group of 1—3 carbon atoms; and optionally thereafter

i) removing an ester if present,
ii) forming an ester,
iii) forming a pharmaceutically acceptable addition salt of an ester.

4. A process for the preparation of a compound of the formula (II) as defined in claim 1 or its ester or acid addition salt thereof which process comprises the hydrogenation of a compound of the formula (V) or ester thereof, or the reaction of a compound of the formula (VII) with a compound of the formula (VI) or ester thereof, and optionally thereafter:

i) acylating the compound of the formula (II) or its ester with a compound of the formula (IX),
ii) hydrogenating the compound of the formula (VIII),
iii) removing an ester if present,
iv) forming an ester,
v) forming a pharmaceutically acceptable acid addition salt of an ester.

5. A process as claimed in claim 1 wherein $R^3$ is a phenyl group.

6. A process as claimed in either claim 1 or claim 5 wherein the compound of the formula (V) is in the form of a hydrogenolysable ester.

7. A process as claimed in any of claims 1—6 adapted to the preparation of a compound of the formula (II) in zwitterionic form.

8. A process for the preparation of a composition which comprises bringing into association a compound of the formula (II) and a pharmaceutically acceptable carrier.

9. A process as claimed in claim 8 which also comprises a penicillin or cephalosporin.

**Patentansprüche für die Vertragsstaaten: BE CH/LI DE FR GB IT LU NL SE**

1. Die Verbindung der Formel (II) oder ein Ester oder ein pharmakologisch verträgliches Säureadditionssalz desselben:

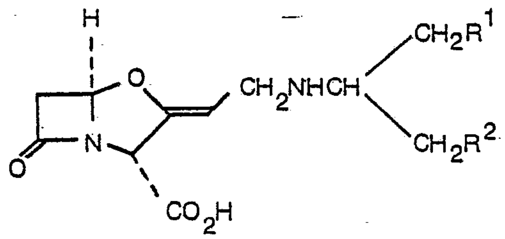

(II)

in der $R^1$ ein Wasserstoffatom oder eine $C_{1-4}$-Alkylgruppe ist und $R^2$ ein Wasserstoffatom oder eine $C_{1-4}$-Alkylgruppe ist oder $R^1$ und $R^2$ zusammen eine Bindung oder eine $-(CH_2)_n-$ Gruppe sein können in der n eine ganze Zahlvon 1 bis 6 ist.

2. Eine Verbindung wie in Anspruch 1 beansprucht, ausgewählt aus
9-N-Isopropylaminodesoxyclavulansäure,
9-N-Cyclopropylaminodesoxyclavulansäure,
9-N-Cyclobutylaminodesoxyclavulansäure,
9-N-Cyclopentylaminodesoxyclavulansäure, und
9-N-Cyclohexylaminodesoxyclavulansäure.

3. Eine Verbindung wie entweder in Anspruch 1 oder Anspruch 2 beansprucht in kristalliner Form.

4. Eine Verbindung wie in Anspruch 1 beansprucht in Form einer freien Säure.

5. Ein Ester wie in Anspruch 1 beansprucht, wobei der Ester die Unterformel $A_1$ oder $CHA_2A_3$ hat:

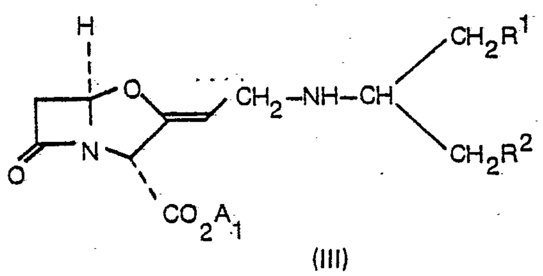

(III)

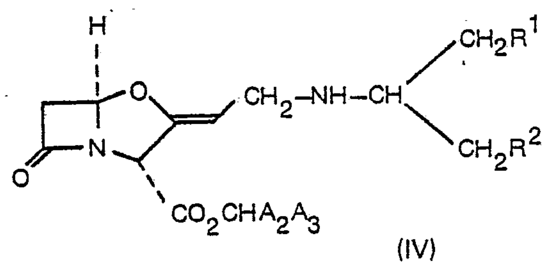

(IV)

in denen $A_1$ eine $C_{1-6}$-Alkylgruppe ist, die gegebenenenfalls mit Alkoxy-, Aryloxy-, Alkoxycarbonyloxy-, Acyloxy-, Arylalkanoyl-, Alkanoyl- oder Benzoylgruppen mit bis zu 8 Kohlenstoffatomen substituiert ist, wobei etwaige Phenylgruppen in solchen Substituenten selbst gegebenenfalls mit einem Fluor-, Brom-, Chloratom, einer Nitro-, $C_{1-4}$-Alkyl- oder $C_{1-4}$-Alkylgruppe substituiert sind; oder eine Phtalidylgruppe ist, die gegebenenfalls mit einer oder 2 Alkyl- oder Alkoxygruppen mit bis zu 3 Kohlenstoffatomen substituiert ist; $A_2$ eine Alkenyl- oder Alkinylgruppe mit bis zu Kohlenstoffatomen oder eine Phenylgruppe ist, die gegebenenfalls mit einem Fluor-, Chlor-, Bromatom, einer Nitro- oder Alkyl- oder Alkoxygruppe mit bis zu 4 Kohlenstoffatomen substituiert ist; und $A_3$ ein Wasserstoffatom, eine Alkylgruppe mit bis zu 4 Kohlenstoffatomen oder eine Phenylgruppe ist, die gegebenenfalls mit einem Fluor-, Chlor-, Bromatom, einer Nitro- oder Alkyl- oder Alkoxygruppe mit bis zu 4 Kohlenstoffatomen substituiert ist.

6. Ein Ester wie entweder in Anspruch 1 oder Anspruch 5 beansprucht in Form eines Säureadditionssalzes.

7. Eine pharmazeutische Zusammensetzung, die eine Verbindung wie in einem der Ansprüche 1 bis 6 beansprucht und einen pharmakologisch verträglichen Träger enthalt.

8. Eine Zusammensetzung wie in Anspruch 7 beansprucht, die auch ein Penicillin oder ein Cephalosporin enthält.

9. Ein Verfahren zur Herstellung einer Verbindung der Formel (II) oder deren Ester oder eines pharmakologisch verträglichen Säureadditionssalzes desselben, umfassend die Hydrierung einer Verbindung der Formel (V):

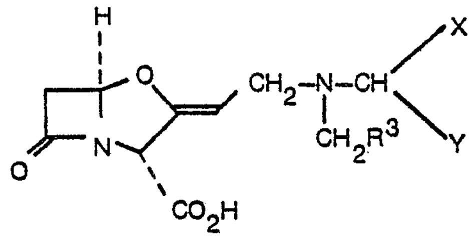

(V)

oder eines Ester derselben, in der X eine —$CH_2R^1$-Gruppe oder eine Gruppe ist, die bei der Hydrierung die —$CH_2R^1$-Gruppe ergibt, und Y eine —$CH_2R^2$-Gruppe oder eine Gruppe ist, die bei der Hydrierung die $CH_2R^2$-Gruppe ergibt, wobei $R^1$ und $R^2$ wie in Anspruch 1 definiert sind, und $R^3$ ein Rest $CR^4$=$CHR^5$ ist, in dem $R^4$ ein Wasserstoffatomen oder eine $C_{1-4}$-Alkylgruppe ist, und $R^5$ eine Wasserstoffatomen, eine $C_{1-4}$-Alkylgruppe oder eine Phenylgruppe ist, die gegebenenfalls inert substituiert ist, oder $R^3$ eine Phenylgruppe ist; und gegebenenfalls anschließend die Durchführung eines oder mehrerer der folgenden Schritte:

i) Entfernen eines Esters, wenn vorhanden,
ii) Bilden eines Esters,
iii) Bilden eines pharmakologisch verträglichen Säureadditionssalzes eines Esters.

10. Ein Verfahren zur Herstellung einer Verbindung der Formel (II) oder deren Ester oder eines Säureadditionssalzes desselben, umfassend die Reaktion einer Verbindung der Formel (VI) oder eines Derivats derselben, das eine Alkylierung gestattet:

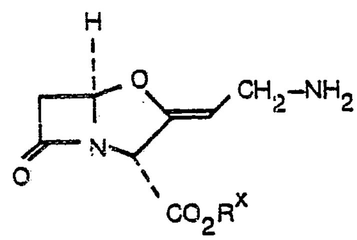

(VI)

in der $R^x$ ein Wasserstoffatom oder eine Estergruppe ist; mit einer Verbindung der Formel (VII):

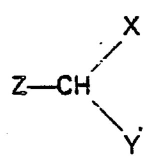

(VII)

in der X und Y wie in bezug auf Formel (V) definiert sind und Z eine leicht verdrängbare Gruppe ist; und gegebenenfalls anschließend:

i) Entfernen eines Esters, wenn vorhanden,
ii) Bilden eines Esters,
iii) Bilden eines pharmakologisch verträglichen Säureadditionssalzes eines Esters,
iv) Umwandeln etwaiger Gruppen X und Y in $CH_2R^1$— und $CH_2R^2$-Gruppen.

11. Ein Verfahren zur Herstellung einer Verbindung der Formel (II) oder deren Ester oder eines Säureadditionssalzes desselben, umfassend die Hydrierung einer Verbindung der Formel (VIII):

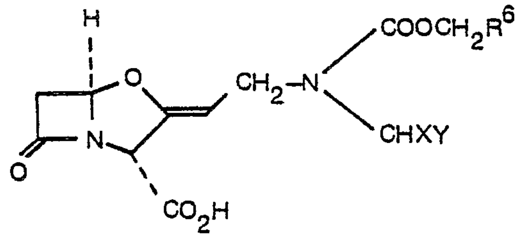

(VIII)

oder eines Ester derselben, in der X und Y wie in bezug auf Formel (V) definiert sind und $R^6$ ein Rest der Unterformel a) ist:

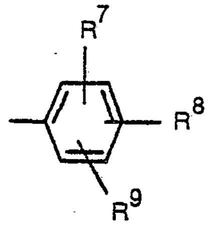

a)

in der $R^7$ ein Wasserstoff- Fluor-, Chlor- und Bromatom oder eine Alkylgruppe mit 1 bis 3 Kohlenstoffatomen, eine Alkoxygruppe mit 1 bis 3 Kohlenstoffatomen, eine Acyloxygruppe mit 1 bis 3 Kohlenstoffatomen, eine Hydroxylgruppe, eine Alkoxycarbonylgruppe mit 1 bis 3 Kohlenstoffatomen im Alkoxyrest, oder eine Gruppe $-N(R^{10})COR^{11}$, $-N(R^{10})SO_2R^{11}$ oder $-CONR^{10}R^{11}$ ist, in der $R^{10}$ ein Wasserstoffatom oder eine Alkylgruppe mit 1 bis 3 Kohlenstoffatomen oder eine Phenyl- oder Benzylgruppe ist, und $R^{11}$ eine Alkylgruppe mit 1 bis 3 Kohlenstoffatomen oder eine Phenyl- oder Benzylgruppe ist; $R^8$ ein Wasserstoff-, Fluor- oder Chloratom oder eine Alkylgruppe mit 1 bis 3 Kohlenstoffatomen, eine Alkoxygruppe mit 1 bis 3 Kohlenstoffatomen oder eine A cyloxygruppe mit 1 bis 3 Kohlenstoffatomen ist; und $R^9$ ein Wasserstoff-, Fluor- oder Chloratom oder eine Alkylgruppe mit 1 bis 3 Kohlenstoffatomen oder eine Alkoxygruppe mit 1 bis 3 Kohlenstoffatomen ist; und gegebenenfalls anschließend

i) Entfernen eines Esters, wenn vorhanden,
ii) Bilden eines Esters,
iii) Bilden eines pharmakologisch verträglichen Säureadditionssalzes eines Esters.

12. Ein verfahren zur Herstellung einer Verbindung der Formel (II) oder ihres Esters oder eines Säureadditionssalzes desselben, umfassend die Hydrierung einer Verbindung der Formel (V) oder eines Esters derselben oder die Reaktion einer Verbindung der Formel (VII) mit einer Verbindung der Formel (VI) oder eines Esters derselben, und gegebenenfalls anschließend:

i) Acylieren der Verbindung der Formel (II) oder deren Ester mit einer Verbindung der Formel (IX),
ii) Hydrieren der Verbindung der Formel (VIII),
iii) Entfernen eines Esters, wenn vorhanden,
iv) Bilden eines Esters,
v) Bilden eines pharmakologisch verträglichen Säureadditionssalzes eines Esters.

**Patentansprüche für den Vertragsstaat: AT**

1. Ein Verfahren zur Herstellung einer Verbindung der Formel (II) oder eines Esters oder eines pharmakologisch verträglichen Säureadditionssalzes desselben:

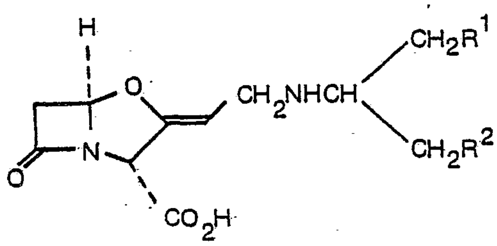

(II)

in der $R^1$ ein Wasserstoffatom oder eine $C_{1-4}$-Alkylgruppe ist und $R^2$ ein Wasserstoffatom oder eine $C_{1-4}$-Alkylgruppe ist oder $R^1$ und $R^2$ zusammen eine Bildung oder eine $-(CH_2)_n-$ Gruppe darstellen können, in der n eine ganze Zahl von 1 bis 6 ist, umfassend die Hydrierung einer Verbindung der Formel (V):

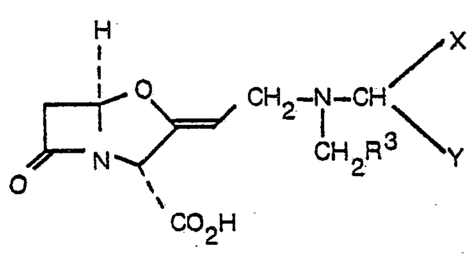

(V)

oder eines Esters derselben, in der X eine —$CH_2R^1$-Gruppe, oder eine Gruppe ist, die bei der Hydrierung die $CH_2R^1$-Gruppe ergibt und Y eine —$CH_2R^2$-Gruppe oder eine Gruppe ist, die bei der Hydrierung die $CH_2R^2$-Gruppe ergibt, wobei $R^1$ und $R^2$ wie in bezug auf Formel (I) oben definiert sind, und $R^3$ ein Rest $CR^4{=}CHR^5$ ist, in dem $R^4$ ein Wasserstoffatom oder eine $C_{1-4}$-Alkylgruppe ist und $R^5$ ein Wasserstoffatom, eine $C_{1-4}$-Alkylgruppe oder eine Phenylgruppe ist, die gegebenenfalls inert substituiert ist, oder $R^3$ eine Phenylgruppe ist; und gegebenenfalls anschließend die Durchführung eines oder mehrerer der folgenden Schritte:

i) Entfernen eines Esters, wenn vorhanden,
ii) Bilden eines Esters,
iii) Bilden eines pharmakologisch verträglichen Säureadditionssalzes eines Esters.

2. Ein Verfahren zur Herstellung einer Verbindung der Formel (II), wie in Anspruch 1 definiert, oder deren Ester oder eines Säureadditionssalzes desselben, umfassend die Reaktion einer Verbindung der Formel (VI) oder eines Derivats derselben, das die Alkylierung gestattet:

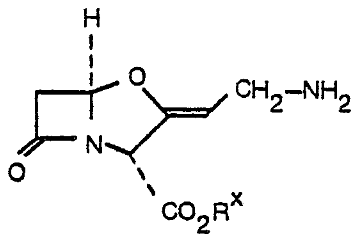

(VI)

in der $R^x$ ein Wasserstoffatom oder eine Estergruppe ist, mit einer Verbindung der Formel (VII):

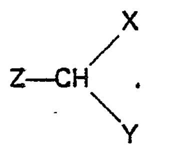

(VII)

in der X und Y wie in bezug auf Formel (V) definiert sind und Z eine leicht verdrängbare Gruppe ist; und gegebenenfalls anschließend:

i) Entfernen eines Ester, wenn vorhanden,
ii) Bilden eines Esters,
iii) Bilden eines pharmakologisch verträglichen Säureadditionssalzes eines Esters.
iv) Umwandeln etwaiger Gruppen X und Y in $CH_2R^1$— und $CH_2R^2$-Gruppen.

3. Ein Verfahren zur Herstellung einer Verbindung der Formel (II) wie in Anspruch 1 definiert oder deren Ester oder eines Säureadditionssalzes desselben, umfassend die Hydrierung einer Verbindung (VIII):

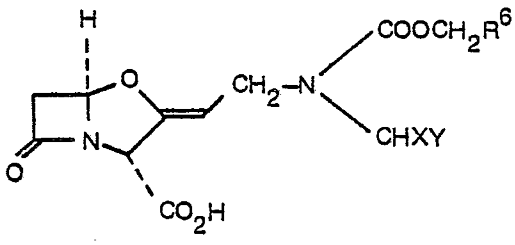

(VIII)

oder eines Esters derselben, in der X und Y wie in bezug auf Formel (V) definiert sind und $R^6$ ein Rest der Unterformel a) ist:

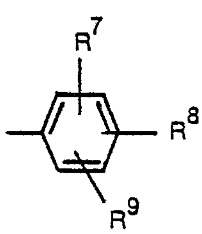

a)

in der $R^7$ ein Wasserstoff-, Fluor-, Chlor- oder Bromatom oder eine Alkylgruppe mit 1 bis 3 Kohlenstoffatom, eine Alkoxygruppe mit 1 bis 3 Kohlenstoffatomen, eine Acyloxygruppe mit 1 bis 3 Kohlenstoffatomen, eine Hydroxylgruppe, eine Alkoxycarbonylgruppe mit 1 bis 3 Kohlenstoffatomen im Alkoxyrest, oder eine Gruppe $—N(R^{10})COR^{11}$, $—N(R^{10})SO_2R^{11}$ oder $—CO—NR^{10}R^{11}$ ist, wobei $R^{10}$ ein Wasserstoffatom oder eine Alkylgruppe mit 1 bis 3 Kohlenstoffatomen oder eine Phenyl- oder Benzylgruppe ist und $R^{11}$ eine Alkylgruppe mit 1 bis 3 Kohlenstoffatomen oder eine Phenyl- oder Benzylgruppe ist; $R^8$ ein Wasserstoff-, Fluor- oder Chloratom oder eine Alkylgruppe mit 1 bis 3 Kohlenstoffatomen eine Alkoxygruppe mit 1 bis 3 Kohlenstoffatomen oder eine Acyloxygruppe mit 1 bis 3 Kohlenstoffatomen ist; und $R^9$ ein Wasserstoff-, Fluor- oder Chloratom oder eine Alkylgruppe mit 1 bis 3 Kohlenstoffatomen oder ein Alkoxygruppe mit 1 bis 3 Kohlenstoffatomen ist; und gegebenenfalls anschließend

i) Entfernen eines Esters, wenn vorhanden,
ii) Bilden eines Esters,
iii) Bilden eines pharmakologisch verträglichen Säureadditionssalzes eines Esters.

4. Ein Verfahren zur Herstellung einer Verbindung der Formel (II), wie in Anspruch 1 definiert, oder deren Ester oder eines Säureadditionssalzes desselben, umfassend die Hydrierung einer Verbindung der Formel (V) oder eines Esters derselben oder die Reaktion einer Verbindung der Formel (VII) mit einer Verbindung der Formel (VI) oder eines Esters derselben, und gegebenenfalls anschließend:

i) Acylieren der Verbindung der Formel (II) oder deren Ester mit einer Verbindung der Formel (IX),
ii) Hydrieren der Verbindung der Formel (VIII),
iii) Entfernen eines Esters, wenn vorhanden,
iv) Bilden eines Esters,
v) Bilden eines pharmakologisch verträglichen Säureadditionssalzes eines Esters.

5. Ein Verfahren wie in Anspruch 1 beansprucht, wobei $R^3$ eine Phenylgruppe ist.

6. Ein Verfahren wie in entweder Anspruch 1 oder Anspruch 5 beansprucht, wobei die Verbindung der Formel (V) in Form eines hydrierbaren Esters ist.

7. Ein Verfahren wie in einem der Ansprüche 1 bis 6 beansprucht, angepaßt zur Herstellung einer Verbindung der Formel (II) in Zwitterionenform.

8. Ein Verfahren zur Herstellung einer Zusammensetzung, das das Zur-Vereinigung-Bringen einer Verbindung der Formel (II) und eines pharmakologisch verträglichen Trägers umfaßt.

9. Ein Verfahren wie in Anspruch 8 beansprucht, das auch ein Penicillin oder Cephalosporin umfaßt.

**Revendications pour les Etats Contractants: BE CH LI DE FR GB IT LU NL SE**

1. Composé de formule (II) ou ester ou sel d'addition avec un acide pharmaceutiquement acceptable de celui-ci:

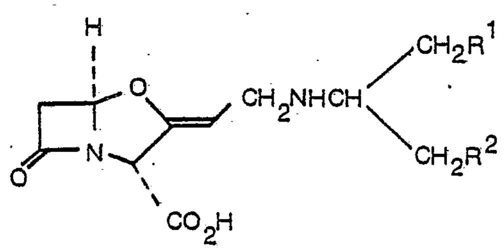

(II)

dans laquelle $R^1$ est un atome d'hydrogène ou un groupe alcoyle en $C_{1-4}$ et $R^2$ est un atome d'hydrogène ou un groupe alcoyle en $C_{1-4}$; ou bien $R^1$ et $R^2$ peuvent représenter ensemble une liaison ou un groupe $—(CH_2)_n—$, où n est un nombre entier allant de 1 à 6.

2. Composé suivant la revendication 1, choisi parmi les suivants:
Acide 9-N-isopropylaminodésoxyclavulanique,
Acide 9-N-cyclopropylaminodésoxyclavulanique,
Acide 9-N-cyclobutylaminodésoxyclavulanique,
Acide 9-N-cyclopentylaminodésoxyclavulanique, et
Acide 9-N-cyclohexylaminodésoxyclavulanique.

3. Composé suivant la revendication 1 ou 2, lorsqu'il se trouve sous la forme cristalline.

4. Composé suivant la revendication 1, lorsqu'il se trouve sous la forme d'un acide libre.

5. Ester suivant la revendication 1, caractérisé en ce que l'ester correspond à la sous-formule $A_1$ or $CHA_2A_3$:

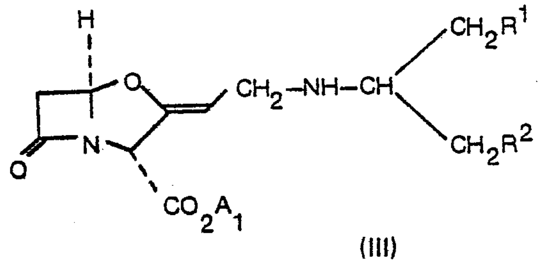

(III)

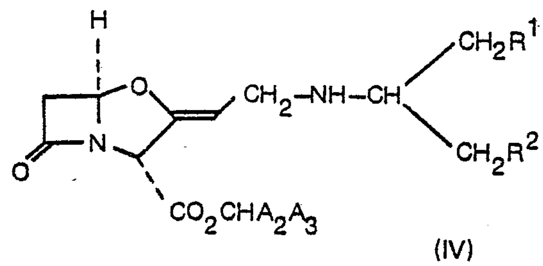

(IV)

dans laquelle $A_1$ est un alcoyle en $C_{1-6}$ facultativement substitué par un groupe alcoxy, aryloxy, alcoxycarbonyloxy, acyloxy, aralcanoyle, alcanoyle ou benzoyle ayant jusqu'à 8 atomes de carbone, n'importe quels groupes phényle dans ces substituants étants eux-mêmes facultativement substitués par un atome de fluor, de brome ou de chlore ou par un groupe nitro, alcoyle en $C_{1-4}$ ou alcoxy en $C_{1-4}$; ou est un groupe phtalidyle facultativement substitué par un ou deux groupes alcoyle ou alcoxy ayant jusqu'à 3 atomes de carbone; $A_2$ est un groupe alcényle ou alcynyle ayant jusqu'à 5 atomes de carbone ou est un groupe phényle facultativement substitué par un atome de fluor, de chlore ou de brome ou par un groupe nitro ou alcoyle ou alcoxy ayant jusqu'à 4 atomes de carbone; et $A_3$ est un atome d'hydrògene, un groupe alcoyle ayant jusqu'à 4 atomes de carbone ou un groupe phénylé éventuellement substitué par un atome de fluor, de chlore ou de brome ou par un groupe nitro ou alcoyle ou alcoxy ayant jusqu'à 4 atomes de carbone.

6. Ester suivant la revendication 1 ou 5, sous la forme d'un sel d'addition avec un acide.

7. Composition pharmaceutique constituée par un composé suivant l'une quelconque des revendications 1 à 6 et par un véhicule ou excipient pharmaceutiquement acceptable.

8. Composition suivant la revendication 7, renfermant également une pénicilline ou une céphalosporine.

9. Procédé pour la préparation d'un composé de formule (II) ou de son ester ou d'un sel d'addition avec un acide pharmaceutiquement acceptable de celui-ci, ce procédé consistant à effectuer l'hydrogénation d'un composé de formule (V):

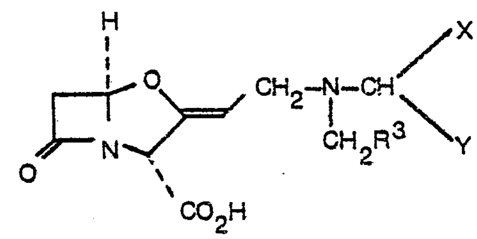

(V)

ou d'un ester de celui-ci, formule dans laquelle X est un groupe $-CH_2R^1$ ou un groupe qui, par hydrogénation, donne le groupe $CH_2R^1$, et Y est un groupe $-CH_2R^2$ ou un groupe qui, par hydrogénation, donne le groupe $CH_2R^2$, où $R^1$ et $R^2$ sont tels que définis dans la revendication 1, et $R^3$ est une fraction molaire $CR^4{=}CHR^5$ dans laquelle $R^4$ est un atome d'hydrogène ou un group alcoyle en $C_{1-4}$ et $R^5$ est un atome d'hydrogène, un groupe alcoyle en $C_{1-4}$ ou un groupe phényle facultativement substitué de façon inerte, ou bien $R^3$ est un groupe phényle; puis facultativement à effectuer un ou plusieurs des stades opératoires ci-après:

i) élimination d'un ester s'il est présent,
ii) formation d'un ester,
iii) formation d'un sel d'addition avec un acide pharmaceutiquement acceptable d'un ester.

10. Procédé pour la préparation d'un composé de formule (II) ou de son ester ou sel d'addition avec un acide de celui-ci, ce procédé consistant à effectuer la réaction d'un composé de formule (VI) ou d'un dérivé de celui-ci qui permet à l'alcoylation de s'effectuer:

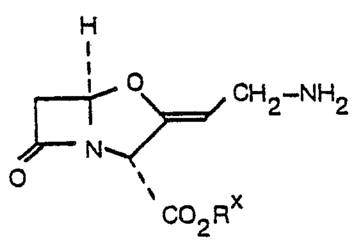

(VI)

dans laquelle $R^x$ est un atome d'hydrogène ou un groupe ester, avec un composé de formule (VII):

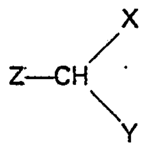

(VII)

dans laquelle X et Y sont tels que définis dans le cas de la formule (V) et Z est un groupe aisément déplaçable, puis facultativement:

i) à éliminer un ester s'il est présent,
ii) à former un ester,
iii) à former un sel d'addition avec un acide pharmaceutiquement acceptable d'un ester,
iv) à convertir les groupes quelconques X et Y en groupes $CH_2R^1$ et $CH_2R^2$.

11. Procédé pour la préparation d'un composé de formule (II) ou de son ester ou sel d'addition avec un acide de celui-ci, ce procédé consistant à effectuer l'hydrogénation d'un composé de formule (VIII):

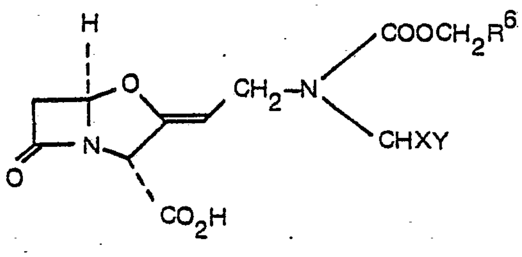

(VIII)

ou d'un ester de celui-ci, formule dans laquelle X et Y sont tels que définis dans le cas de la formule (V), et $R^6$ est une fraction molaire de de sous-formule (a):

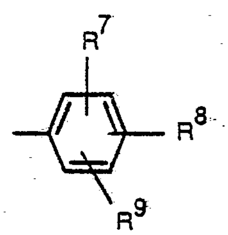

a)

dans laquelle $R^7$ est un atome d'hydrogène, de fluor, de chlore ou de brome ou un groupe alcoyle ayant de 1 à 3 atomes de carbon, un groupe alcoxy ayant de 1 à 3 atomes de carbone, un groupe acyloxy ayant de 1 à 3 atomes de carbone, un groupe hydroxy ou un groupe alcoxycarbonyle contenant de 1 à 3 atomes de carbone dans la fraction alcoxy, ou bien un groupe —$N(R^{10})CO.R^{11}$, —$N(R^{10})SO_2R^{11}$ ou —CO—$NR^{10}R^{11}$, où $R^{10}$ est un atome d'hydrogène ou un groupe alcoyle ayant de 1 à 3 atomes de carbone, ou bien un groupe phényle ou benzyle, et $R^{11}$ est un groupe alcoyle ayant de 1 à 3 atomes de carbone ou un groupe phényle ou benzyle; $R^8$ est un atome d'hydrogène, de fluor ou de chlore ou un groupe alcoyle ayant de 1 à 3 atomes de carbone, un groupe alcoxy ayant de 1 à 3 atomes de carbone ou un groupe acyloxy ayant de 1 à 3 atomes de carbone; et $R^9$ est un atome d'hydrogène, de fluor ou de chlore ou un groupe alcoyle ayant de 1 à 3 atomes de carbone ou un groupe alcoxy ayant de 1 à 3 atomes de carbone; puis facultativement:

i) à éliminer un ester s'il est présent,
ii) à former un ester,
iii) à former un sel d'addition avec un acide pharmaceutiquement acceptable d'un ester.

12. Procédé pour la préparation d'un composé de formule (II) ou de son ester ou du sel d'addition avec un acide de celui-ci, ce procédé consistant à effectuer l'hydrogénation d'un composé de formule (V) ou de son ester, ou la réaction d'un composé de formule (VII) avec un composé de formule (VI) ou son ester, puis facultativement:

i) à acyler le composé de formule (II) ou son ester avec un composé de formule (IX),
ii) à hydrogéner le composé de formule (VIII),

iii) à éliminer un ester s'il est présent,
iv) à former un ester,
v) à former un sel d'addition avec un acide pharmaceutiquement acceptable d'un ester.

**Revendications pour l'Etat Contractant: AT**

1. Procédé pour la préparation d'un composé de formule (II) ou d'un ester ou sel d'addition avec un acide pharmaceutiquement acceptable de celui-ci:

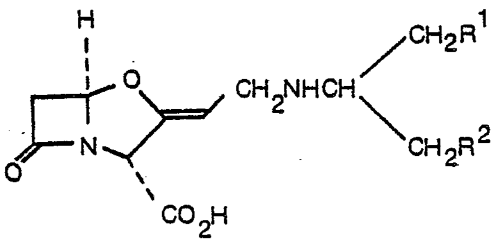

(II)

dans laquelle $R^1$ est un atome d'hydrogène ou un groupe alcoyle en $C_{1-4}$ et $R^2$ est un atome d'hydrogène ou un groupe alcoyle en $C_{1-4}$; ou bien $R^1$ et $R^2$ peuvent représenter ensemble une liaison ou un groupe $-(CH_2)_n-$, où n est un nombre entier allant de 1 à 6, ce procédé consistant à effectuer l'hydrogénation d'un composé de formule (V):

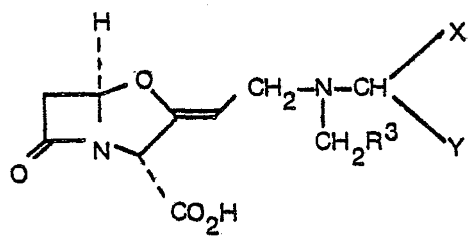

(V)

ou d'un ester de celui-ci, formule dans laquelle X est un groupe $-CH_2R^1$ ou un groupe qui, par hydrogénation, donne le groupe $CH_2R^1$, et Y est un groupe $-CH_2R^2$ ou un groupe qui, par hydrogénation, donne le groupe $CH_2R^2$, où $R^1$ et $R^2$ sont tels que définis pour la formule (I) ci-dessus, et $R^3$ est une fraction molaire $CR^4{=}CHR^5$ dans laquelle $R^4$ est un atome d'hydrogène ou un groupe alcoyle en $C_{1-4}$ et $R^5$ est un atome d'hydrogène, un groupe alcoyle en $C_{1-4}$ ou un groupe phényle facultativement substitué de façon inerte, ou bien $R^3$ est un groupe phényle; puis facultativement à effectuer un ou plusieurs des stades opératoires ci-après:

i) élimination d'un ester s'il est présent,
ii) formation d'un ester,
iii) formation d'un sel d'addition avec un acide pharmaceutiquement acceptable d'un ester.

2. Procédé pour la préparation d'un composé de formule (II) suivant la revendication 1, ou de son ester ou sel d'addition avec un acide de celui-ci, ce procédé consistant à effectuer la réaction d'un composé de formule (VI) ou d'un dérivé de celui-ci qui permet à l'alcoylation de s'effectuer:

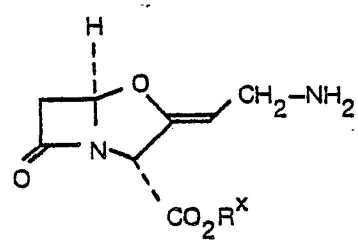

(VI)

dans laquelle $R^x$ est un atome d'hydrogène ou un groupe ester, avec un composé de formule (VII):

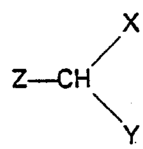

(VII)

dans laquelle X et Y sont tels que définis dans le cas de la formule (V) et Z est un groupe aisément déplaçable, puis facultativement:

i) à éliminer un ester s'il est présent,
ii) à former un ester,
iii) à former un sel d'addition avec un acide pharmaceutiquement acceptable d'un ester,
iv) à convertir les groupes quelconques X et Y en groupe $CH_2R^1$ et $CH_2R^2$.

3. Procédé pour la préparation d'un composé de formule (II) suivant la revendication 1 ou de son ester ou sel d'addition avec un acide de celui-ci, ce procédé consistant à effectuer l'hydrogénation d'un composé de formule (VIII):

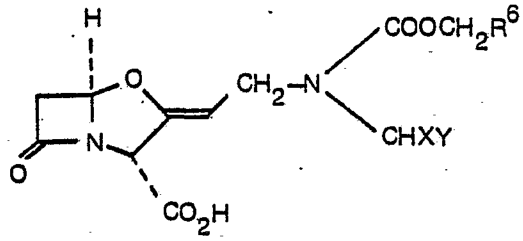

(VIII)

ou d'un ester de celui-ci, formule dans laquelle X et Y sont tels que définis dans le cas de la formule (V), et $R^6$ est une fraction molaire de sous-formule (a):

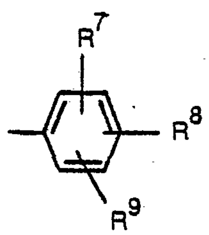

a)

dans laquelle $R^7$ est un atome d'hydrogène, de fluor, de chlore ou de brome ou un groupe alcoyle ayant de 1 à 3 atomes de carbone, un groupe alcoxy ayant de 1 à 3 atomes de carbone, un groupe acyloxy ayant de 1 à 3 atomes de carbone, un groupe hydroxy ou un groupe alcoxycarbonyle contenant de 1 à 3 atomes de carbone dans la fraction alcoxy, ou bien un group $-N(R^{10})CO.R^{11}$, $-N(R^{10})SO_2R^{11}$ ou $-CO-NR^{10}R^{11}$, où $R^{10}$ est un atome d'hydrogène ou un groupe alcoyle ayant de 1 à 3 atomes de carbone, ou bien un groupe phényle ou benzyle, et $R^{11}$ est un groupe alcoyle ayant de 1 à 3 atomes de carbone ou un groupe phényle ou benzyle; $R^8$ est un atome d'hydrogène, de fluor ou de chlore ou un groupe alcoyle ayant de 1 à 3 atomes de carbone, un groupe alcoxy ayant de 1 à 3 atomes de carbone ou un groupe acyloxy ayant de 1 à 3 atomes de carbone; et $R^9$ est un atome d'hydrogène, de fluor ou de chlore ou un groupe alcoyle ayant de 1 à 3 atomes de carbone ou un groupe alcoxy ayant de 1 à 3 atomes de carbone; puis facultativement:

i) à éliminer un ester s'il est présent,
ii) à former un ester,
iii) à former un sel d'addition avec un acide pharmaceutiquement acceptable d'un ester.

4. Procédé pour la préparation d'un composé de formule (II) suivant la revendication 1 ou de son ester ou du sel d'addition avec un acide de celui-ci, ce procédé consistant à effectuer l'hydrogénation d'un composé de formule (V) ou de son ester, ou la réaction d'un composé de formule (VII) avec un composé de formule (VI) ou son ester, puis facultativement:

i) à acyler le composé de formule (II) ou son ester avec un composé de formule (IX),
ii) à hydrogéner le composé de formule (VIII),
iii) à éliminer un ester s'il est présent,
iv) à former un ester,
v) à former un sel d'addition avec un acide pharmaceutiquement acceptable d'un ester.

5. Procédé suivant la revendication 1, caractérisé en ce que $R^3$ est un groupe phényle.

6. Procédé suivant la revendication 1 ou 5, caractérisé en ce que le composé de formule (V) est sous la forme d'un ester hydrogénolysable.

7. Procédé suivant l'une quelconque des revendications 1 à 6, adapté à la préparation d'un composé de formule (II) sous la forme du zwittérion.

8. Procédé pour la préparation d'une composition consistant à associer un composé de formule (II) et un véhicule ou excipient pharmaceutiquement acceptable.

9. Procédé suivant la revendication 8, qui utilise également une pénicilline ou une céphalosporine.